(19) Europäisches Patentamt
European Patent Office
Office européen des brevets

(11) Publication number: **0 112 620 B1**

(12) **EUROPEAN PATENT SPECIFICATION**

(45) Date of publication of patent specification: **30.12.86**

(21) Application number: **83306459.5**

(22) Date of filing: **25.10.83**

(51) Int. Cl.$^4$: **C 07 D 233/76,** C 07 D 233/78, C 07 D 407/12, A 61 K 31/415, A 61 K 31/34

# (54) 5-(Substituted phenyl) hydantoins.

(30) Priority: **01.11.82 US 438199**
**01.11.82 US 438200**

(43) Date of publication of application:
**04.07.84 Bulletin 84/27**

(45) Publication of the grant of the patent:
**30.12.86 Bulletin 86/52**

(84) Designated Contracting States:
**AT BE CH DE FR GB IT LI LU NL SE**

(58) References cited:
**GB-A-2 012 756**
**GB-A-2 053 206**

(73) Proprietor: **PFIZER INC.**
**235 East 42nd Street**
**New York, N.Y. 10017 (US)**

(72) Inventor: **Rizzi, James P.**
**34 Devonshire Drive**
**Waterford Connecticut (US)**
Inventor: **Schnur, Rodney C.**
**91 Front Street**
**Noank Connecticut (US)**

(74) Representative: **Graham, Philip Colin Christison et al**
**Pfizer Limited Ramsgate Road**
**Sandwich, Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 620 B1

**Description**

This invention relates to novel 5-(substituted phenyl)hydantoins and to pharmaceutically acceptable salts thereof as inhibitors of aldose reductase useful as therapeutic agents for the treatment of chronic diabetic complications; and to intermediates therefor.

Despite the widespread use of insulin and of the availability of a large number of synthetic hypoglycemic agents such as the sulfonylureas (e.g. chloropropamide, tolbutamide, acetoxhexamide) and biguanides (e.g. phenformin), the search for improved hyproglycemic agents continues. More recently, efforts have been directed to controlling certain chronic complications of diabetes, such as diabetic cataracts, neuropathy and retinopathy. Such efforts have given rise to development of aldose reductase inhibitors, compounds which inhibit the activity of the enzyme aldose reductase which is primarily responsible for regulating reduction of aldoses to the corresponding polyols. In this way, unwanted accumulation of galactitol in the lens of galactosemic subjects and of sorbitol in the lens, kidney and peripheral nervous cord of various diabetic subjects is prevented or reduced. References which described aldose reductase inhibitors are U.S. 3,821,383 — 1,3-dioxo-1H-benz[d,e]isoquinoline-2(3H)-acetic acid and related compounds; U.S. 4,200,642 — spiro-oxazolidine-2,4-diones; U.S. 4,117,230; 4,130,714; 4,147,797; 4,210,756; 4,235,911 and 4,282,229, each of which describes certain spirohydantoins.

U.S. 4,281,009 describes a series of 5,5-disubstituted hydantoins in which one substituent is a substituted phenyl group and the other an alkyl or a heterocyclic group, said compounds being useful for treatment of diseases caused by stress.

Henze et al., J. Am. Chem. Soc. *64*, 522—3 (1942) describe 5-phenylhydantion and certain 5-(mono- and di-substituted phenyl)hydantoins wherein the substituents are hydroxy, alkoxy, formyl, methyl, chloro or dimethylamino. Other 5-(substituted phenyl)-hydantoins are disclosed in U.S. 3,410,865 and in British Patent Application 2,063,206A. None of these known 5-(phenyl)hydantoins are reported to be aldose reductase inhibitors or to have a thio, sulfinyl or sulfonyl substituent in the phenyl ring.

It has now been found that certain 5-(substituted)phenyl hydantoins of formula I below and pharmaceutically acceptable salts thereof are aldose reductose inhibitors useful as therapeutic agents for preventing and/or alleviating chronic diabetic complications.

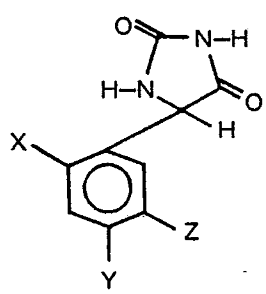

I

In formula (I), each of X and Y is hydrogen, chloro, fluoro, bromo, $(C_1—C_6)$alkyl, $(C_1—C_6)$alkoxy, trifluoromethyl, amino or nitro;

Z is $S(O)_mR$, $SO_2NHR^1$ or $(C_1—C_4)$alkoxy; with the proviso that each of X and Y is fluoro, chloro, $(C_1—C_4)$alkyl or nitro when Z is $(C_1—C_4)$alkoxy;

R is $(C_1—C_6)$alkyl, chloro, methoxymethyl or

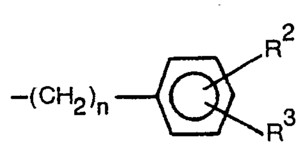

;

m is 0, 1 or 2, with the proviso that when R is chloro m is 2;

n is an integer of from 1 to 4;

each of $R^2$ and $R^3$ is hydrogen, chloro, fluoro, bromo, $(C_1—C_6)$alkyl, $(C_1—C_6)$alkoxy, amino or nitro;

$R^1$ is hydrogen, furfuryl,

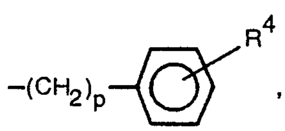

,

$(C_1—C_6)$alkyl, omega-substituted $(C_2—C_6)$alkyl wherein the substituent is hydroxy or dimethylamino; $R^4$ is hydrogen, fluoro or chloro; and p is 0 or an integer of from 1 to 4.

The favored compounds of formula I are those wherein Z is $S(O)_mR$; m is 2; and R is $(C_1—C_6)$alkyl or

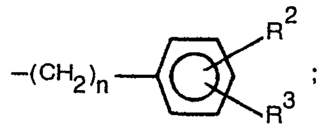

n is 1 or 2; and those wherein Z is $SO_2NHR^1$ wherein $R^1$ is furfuryl or

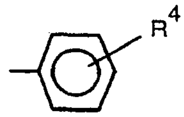

wherein $R^4$ is fluoro or chloro. The preferred compounds are those favored compounds wherein X is fluoro or chloro; Y is hydrogen, fluoro, chloro or methyl, and Z is

$-SO_2CH_3$, 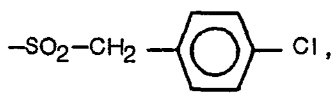, 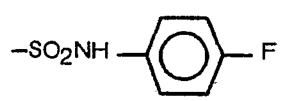 or 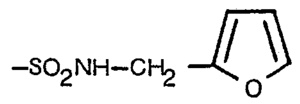.

Other preferred compounds are those wherein Z is methoxy or ethoxy; X is fluoro or chloro and Y is fluoro, chloro or methyl.

Further preferred compounds are those wherein Z is $SCH_3$ or $SCH_2OCH_3$, X is fluoro or chloro; and Y is hydrogen, fluoro, chloro, methyl or trifluoromethyl, or wherein Z is $SO_2Cl$ X is chloro or fluoro and Y is hydrogen, chloro, fluoro or methyl.

The present invention also includes the pharmaceutically acceptable acid addition salts of those compounds of formula I wherein X and or Y is amino. Representative of said salts, but not limited thereto are the hydrochloride, hydrobromide, sulfate, phosphate, nitrate, acetate, lactate, citrate, malate, succinate and gluconate. Such salts are prepared by contacting the free base with the appropriate mineral or organic acid in either aqueous solution or in a suitable organic solvent. The solid salt is then obtained by precipitation or by evaporation of the solvent.

Because of the acidic hydrogen atom in the hydantion ring of the compounds of formula I, salts can be formed with pharmaceutically acceptable cations by conventional methods. Thus, these salts may be readily prepared by treating the compound of formula I with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired metal and the solution subsequently evaporated to dryness. Suitable pharmaceutically acceptable cations for this purpose include, but are not limited to, potassium, sodium, ammonium, calcium and magnesium, aluminum, benzathine, piperazine, N-methylglucamine and procaine.

It is to be understood that by use of the term pharmaceutically-acceptable salts herein it is meant to embrace both the acid addition salts and the salts formed with appropriate cations, as described above.

Also embraced by the present invention are pharmaceutical compositions comprising a pharmaceutically acceptable carrier and a compound of formula I in an amount effective for the treatment of diabetes-associated complications, including diabetic cataracts, neuropathy and retinopathy. Preferred compounds for use in such pharmaceutical compositions are those having the preferred substituents as defined herein above.

The compounds of the present invention are useful for the treatment of diabetes-associated complications, including diabetic cataracts, neuropathy and retinopathy. To carry out such treatment, an effective amount of a compound of formula I, preferably a compound having the preferred substituents for X, as defined herein above, is administered to a subject in need of the treatment.

The compounds of formula I are preferably prepared by a process involving the following reactions:

(1) when Z is —SR or ($C_1$—$C_4$)alkoxy a 2-mercaptobenzaldehyde of the formula:—

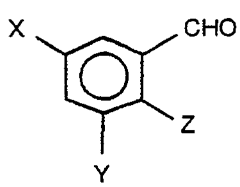

wherein Z is SR or ($C_1$—$C_4$)alkoxy and X, Y and R are as defined above, is reacted with ammonium carbonate and potassium or sodium cyanide in aqueous alcohol solution at a temperature of from 50°C to 60°C;

(2) when Z is —S(O)R, a hydantoin thioether of the formula:—

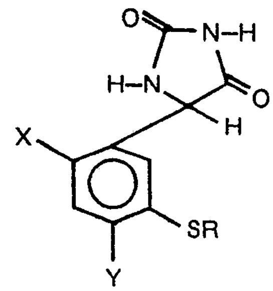

(V)

as formed in step (1), is reacted with sodium periodate in aqueous alcoholic solution at ambient temperature

(3) when Z is —$SO_2R$, a hydantoin thioether of formula (V) is reacted with potassium permanganate in acetic acid at a temperature of from −10°C to room temperature;

(4) when Z is $SO_2Cl$, a hydantoin thioether of formula (V; where R is methoxymethyl, is reacted with chlorine in a reaction-inert solvent;

(5) when Z is $SO_2NHR^1$, a sulfonyl chloride of the formula:—

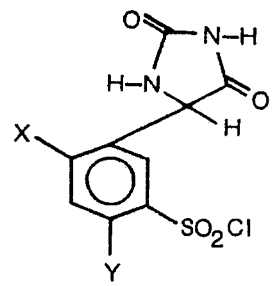

(IX)

as formed in step (4), is reacted with an amine of formula $H_2NR^1$ in a reaction-inert solvent at a temperature of from 0°C to 50°C; and

(6) if desired, the compound of formula (I) is converted into a pharmaceutically-acceptable salt thereof.

The above reactions are illustrated in more detail in the Reaction Schemes which follow. These Reaction Schemes also include the preparation of starting compounds.

The preparation of compounds of this invention wherein Z is $S(O)_mR$ and R is other than chloro is illustrated in Reaction Scheme A:

REACTION SCHEME A

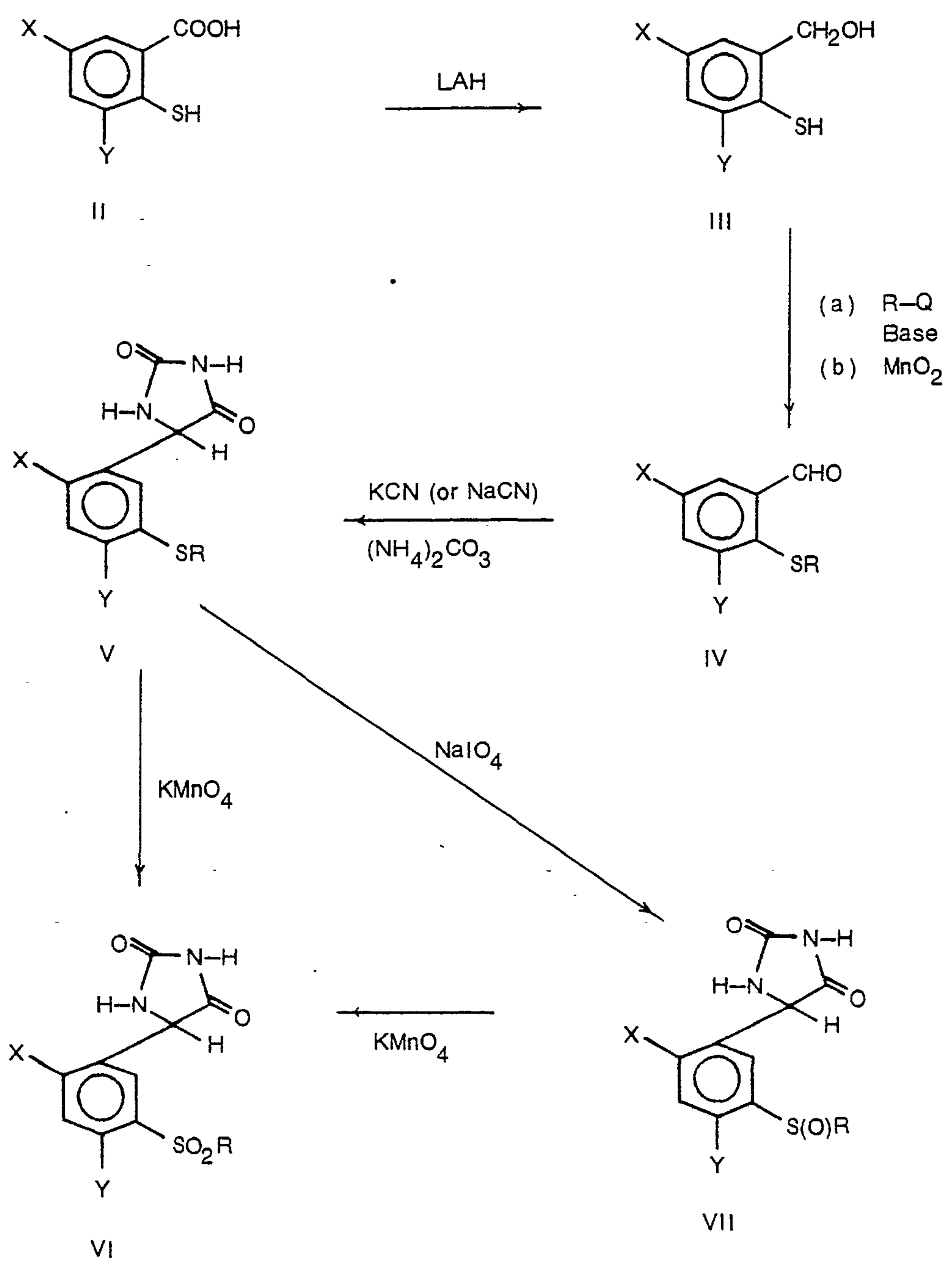


The preparation of compounds of formula I wherein Z is $SO_2Cl$ or $SO_2NHR^1$ is illustrated in Scheme B:

REACTION SCHEME B

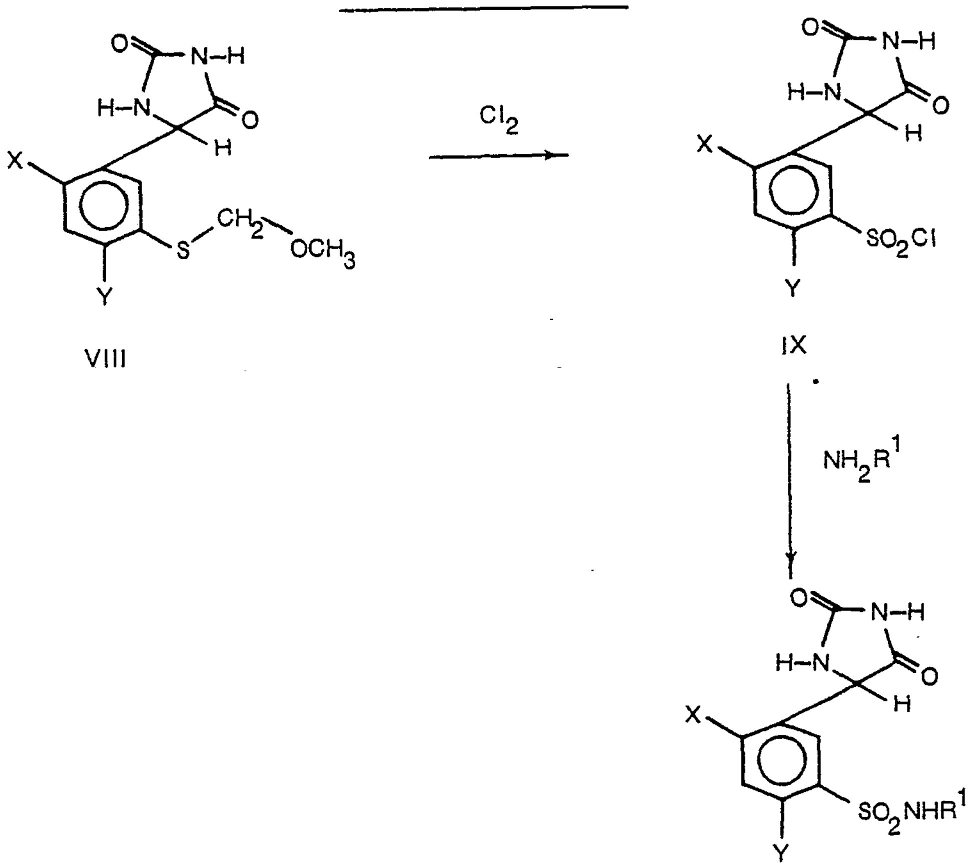


VIII

IX

X

The preparation of compounds of this invention wherein Z is $(C_{1-4})$ alkoxy is illustrated in Reaction Scheme C:

REACTION SCHEME C

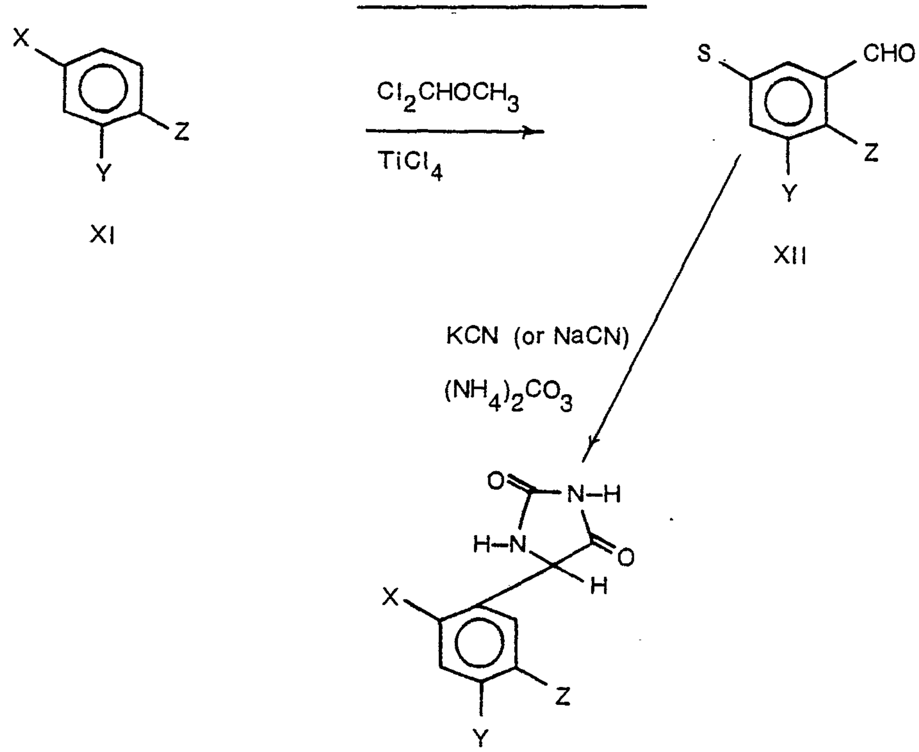


XI

XII

Convenient starting materials for Reaction Scheme A are the appropriate 2-mercaptobenzoic acid derivatives of formula II or the 2-mercaptobenzaldehydes of formula IV. Many of the required benzoic acid or benzaldehyde derivatives are known compounds. Those that are not described in the literature are readily preparable by methods known to those skilled in the art.

In the first step of Scheme A, the appropriate 2-mercaptobenzoic acid (formula II) is reduced to the corresponding 2-mercaptobenzyl alcohol derivative by direct reduction of the carboxyl group using lithium aluminum hydride (LAH). The reduction is conducted in a reaction-inert solvent such as dialkyl ether, a cyclic ether, e.g. diethyl ether, dioxane, tetrahydrofuran, ethyleneglycol dimethyl ether or diethyleneglycol dimethyl ether, at a temperature of from −10°C to +30°C. In general, the compounds to be reduced is added to a solution or slurry of the reagent. An excess of reagent, up to 25% excess, is usually used to ensure complete reduction. The unreacted and/or excess reagent is destroyed by addition of ethyl acetate. The reduction product is isolated by known procedures.

In addition to lithium aluminum hydride, other hydride reducing agents such as diisobutyl aluminum hydride, sodium diethyl aluminum hydride or sodium bis(2-methoxyethoxy)aluminum hydride may be used to reduce the benzoic acid starting material to the corresponding benzyl alcohol.

In the case of each of the above mentioned reducing agents, acid chloride, ester and anhydride derivatives of the 2-mercaptobenzoic acid reagent (II) may be used in place of said 2-mercaptobenzoic acid as starting material.

In the next step, alkylation or aralkylation of the mercapto group is achieved by reacting the 2-mercaptobenzyl alcohol (III) in a reaction-inert solvent with the appropriate alkylating or aralkylating agent R—Q in the presence of a base. The group R is as defined above and Q is chloro, bromo or iodo, or tosylate.

Suitable bases for the alkylation or aralkylation are alkoxides, hydrides and hydroxides of sodium and potassium; organic bases such as trialkylamines, pyridine, dimethylaniline and N-methylmorpholine.

The alkylating or aralkylating agent and the mercaptobenzyl alcohol (III) are reacted in molar ratios ranging from equimolar to up to 10% excess of said agent. Greater excesses of said agent offer no advantage and are generally avoided for reasons of economy. The amount of base used is at least equal to the amount of mercaptobenzyl alcohol reactant. The thus-produced thioether derivative is then oxidized to the corresponding benzaldehyde (IV) by means of activated manganese dioxide in a reaction-inert solvent at temperatures ranging from 20°C to 100°C. In most instances ambient temperature is used. Suitable reaction-inert solvents are methylene chloride, chloroform, acetone, dioxane, tetrahydrofuran, benzene and toluene. An excess of manganese dioxide, generally from 4 to 15 equivalents, is used in order to ensure complete reaction. The reaction period, of course, depends upon the reaction temperature, the amount of oxidizing agent used, and upon the nature of the compound to be oxidized. In general, reaction periods of from 4 hours to 24 hours are required. The oxidized product (IV) is recovered by known methods as by filtration to remove the excess Manganese dioxide and evaporation of the filtrate under reduced pressure to afford the crude benzaldehyde derivative (IV). The benzaldehyde derivative is generally used without further purification in Scheme A.

The benzaldehyde derivative (IV) is converted to the hydantoin (V) by reaction with ammonium carbonate and potassium (or sodium) cyanide in aqueous alcohol solution at a temperature of from 50°—60°C for from 2 to 24 hours. Molar ratios of benzaldehyde reactant: potasium (or sodium) cyanide: ammonium carbonate of from 1:2:4 afford satisfactory yields of the desired hydantoin. The product is recovered by acidifying the reaction mixture and extracting it with a water immiscible solvent such as ethyl acetate.

The hydantoin thioether compound (V) is then oxidized to the corresponding sulfone (VI) directly or stepwise via the intermediate sulfoxide (VII). The stepwise oxidation is carried out by treating the thioether (V) with sodium periodate in aqueous alcoholic solution at ambient temperature using a 1:2 molar ratio of thioether to sodium periodate. The product, a mixture of diastereomeric sulfoxides (VII) is recovered by extraction with a water-immiscible solvent, e.g. ethyl acetate.

Direct oxidation of the thioether (V) to the corresponding sulfonyl derivative (VI) is conveniently carried out by reacting the thioether with potassium permanganate in acetic acid at a temperature of from about −10° to room temperature. Two moles of permanganate are used per mole of thioether. The product is recovered by quenching the reaction mixture in dilute aqueous sodium bisulfite and extraction of the product therefrom with a water-immiscible solvent such as ethyl acetate.

Alternatively, the sulfonyl derivative (VI) is prepared by potassium permanganate oxidation of the sulfoxide (VII). Except for the use of an equimolar amount of permanganate, the procedure is the same as that described for the direct conversion of thioether to sulfonyl derivative.

In Reaction Scheme B, the compound of Formula (IX), which serves as an intermediate for the sulfonamido derivative of formula (X), is prepared from a compound of formula (VIII), which corresponds to formula (V) wherein R is methoxymethyl, by reacting said compound with chlorine in a reaction-inert solvent. In a typical example, chlorine gas is passed into a solution of the methoxymethylthio ether VIII) in a water-miscible solvent-water medium at 0°C to 20°C until a yellow color persists. The reaction mixture is quenched by adding it to dilute aqueous sodium bisulfite and the product recovered therefrom by extraction. Suitable solvents for this reaction are dioxane, tetrahydrofuran, 1,2-dimethoxyethane (monoglyme) and diethyleneglycol dimethyl ether (diglyme).

The sulfonyl chloride of formula (IX) is converted to sulfonamide of formula (X) by reacting it with the appropriate amine of formula $H_2NR^1$ in a reaction-inert solvent at from 0°C to 50°C until reaction is complete. A variety of solvents can be used such as methylene chloride, chloroform, dioxane, tetrahydrofuran, monoglyme, diglyme and alcohols. The product is recovered by quenching the reaction mixture in dilute aqueous acid followed by extraction of the resulting solution with a water-immiscible solvent, such as ethyl acetate.

Convenient starting materials for Reaction Scheme C are the appropriate phenyl alkyl ethers of formula XI or the 2-alkoxy-benzaldehydes of formula XII. Many of the required phenyl alkyl ethers or benzaldehyde derivatives are known compounds. Those that are not described in the literature are readily preparable by methods known to those skilled in the art.

In the first step of Scheme C, the appropriate phenyl alkyl ether (formula XI) is formylated to give the corresponding aldehyde derivative by direct formulation of the benzene ring using alpha, alpha-dichloromethyl methyl ether and titanium tetrachloride. The reaction is conducted in a reaction-inert solvent such as a halo carbon, e.g. dichloromethane, chloroform, dichloroethane or carbon tetrachloride, and at a temperature of from −10°C t +30°C. In general, the compounds to be formylated is added to a solution of the titanium tetrachloride reagent. An excess of reagent, up to 100% excess, is usually used to ensure complete reduction. The formylating agent, dichloromethyl methyl ether is then added dropwise to this reaction mixture. The unreacted and/or excess reagents are destroyed by addition of a large excess of water. The reaction product is isolated by known procedures.

Alternatively, the required benzaldehyde reactant (XII) is made from the appropriate phenol via the Duff reaction which comprises formylation of a phenol via the Duff reaction which comprises formylation of a phenol with hexamethylenetetramine in the presence of an acidic catalyst. The 2-hydroxy benzaldehyde derivatives thus produced is then alkylated via the Williamson reaction to afford the desired 2-alkoxybenzaldehyde reactant (XII).

A still further method for producing the necessary 2-hydroxybenzaldehyde reactant comprises reaction of the appropriate phenol compound; i.e., a 2-Y-4-X-disubstituted-hydroxy benzene, with chloroform in aqueous NaOH (Reimer-Tiemann reaction) to afford the desired 2-hydroxy-3-Y-5-X benzaldehyde, which is alkylated by the method described above.

The benzaldehyde derivative (XII) is converted to the hydantion (I) by reaction with ammonium carbonate and potassium (or sodium) cyanide in aqueous alcohol solution at a temperature of from 50—60°C for from 2 to 24 hours. Molar ratios of benzaldehyde reactant: potassium (or sodium) cyanide:ammonium carbonate of from 1:2:4: afford satisfactory yields of the desired hydantoin. The product is recovered by acidifying the reaction mixture and extracting it with a water-immiscible solvent such as ethyl acetate.

The novel compounds of formula I and the pharmaceutically acceptable salts thereof are useful as inhibitors of the enzyme aldose reductase in the treatment of chronic complications of diabetes, such as diabetic cataracts, retinopathy and neuropathy. Treatment of subjects with the compounds of this invention includes both the prevention and alleviation of such conditions. The compounds may be administered to a subject in need of treatment by a variety of conventional routes of administration, including oral, parenteral and topical. In general, these compounds will be administered orally or parenterally at dosages from 0.25 to 25 mg/kg body weight of the subject to be treated per day, preferably from 1.0 to 10 mg/kg. However, depending on the condition of the subject being treated, some variation in dosage will necessarily occur. The person responsible for administration will, in any event, determine the appropriate dose for the individual subject.

The novel compounds of this invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups or injectable solutions.

The pharmaceutical compositions, if desired, may contain additional ingredients such as flavorings, binders and excipients. Thus, for purposes of oral administration, tablets containing various excipients such as sodium citrate, calcium carbonate or calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type also may be employed as fillers in soft and hard filled gelatin capsules. Preferred materials for this include lactose or milk sugar and high molecular weight polyethylene glycols when aqueous suspensions or elixirs are desired for oral administration, the essential active ingredient therein may be combined with various sweetening or flavoring agents, together with diluents such as water, ethanol, propylene glycol, glycerine and combinations thereof.

For parenteral administration, solutions of the novel compound of formula I in sesame or peanut oil, aqueous propylene glycol, or in sterile aqueous solution can be employed. Such aqueous solutions should be suitably buffered if necessary and the liquid diluent first rendered isotonic with sufficient saline or glucose. These particular aqueous solutions are especially suitable for intravenous, intramuscular,

subcutaneous and intraperitoneal administration. In this connection, the sterile aqueous media employed are all readily available by standard techniques known to those skilled in the art.

The higher solubility of the present compounds of formula I and of the pharmaceutically acceptable salts thereof in aqueous solution, compared to other similar compounds and especially compared to the corresponding compounds of formula I having no amino or substituted amino substituents, is advantageous not only for the preparation of aqueous pharmaceutical compositions for parenteral administration, as described above, but also more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the compounds of this invention are administered to the eye of the subject in need of teatment in the form of an ophthalmic preparation prepared in accordance with conventional pharmaceutical practice, see for example "Remington's Pharmaceutical Sciences" 15th Edition, pages 1488 to 1501 (Mack Publishing Co., Easton, PA). The ophthalmic preparation will contain a compound of formula I or a pharmaceutically acceptable salt thereof in a concentration from about 0.1 to about 5% by weight, preferably from about 0.5 to about 2% in a pharmaceutically acceptable solution, suspension or ointment. Some variation in concentation will necessarily occur, depending on the particular compound employed, the condition of the subject to be treated and the like, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents, viscosity-increasing agents and the like. Suitable preservatives include benzalkonium chloride, benzethonium chloride, chlorobutanol, thimerosal and the like. Suitable buffers include boric acid, sodium and potassium bicarbonate, sodium and potassium borate, sodium and potassium carbonate, sodium acetate, sodium biphosphate and the like, in amounts sufficient to maintain the pH at between about 6 and 8, preferably between about 7 and 7.5. Suitable tonicity agents are dextran 40, dextran 70, dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilzers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxymethylpropylcellulose, lanolin, methylcellulose, petrolatum, polyethylene glycol, polyvinyl alcohol, polyvinylpyrrolidone, carboxymethylcellulose and the like. The ophthalmic preparation will be administered topically to the eye of the subject in need of treatment by conventional methods, for excample in the form of drops or by bathing the eye in the ophthalmic solution.

The activity of the compounds of the present invention as agents for the control of chronic diabetic complications may be determined by a number of standard biological or pharmacological tests. Suitable tests include (1) measuring their ability to inhibit the enzyme activity of isolated aldose reductase; (2) measuring their ability to reduce or inhibit sorbitol accumulation in the sciatic nerve of acutely streptozotocinized (i.e. diabetic) rats; (3) measuring their ability to reverse already elevated sorbitol levels in the sciatic nerve and lens of chronic streptozotocin-induced diabetic rats; (4) measuring their ability to prevent or inhibit galactitol formation in the lense of acutely galactosemic rats; and (5) measuring their ability to delay cataract formation and reduce the severity of lens opacities in chronic galactosemic rats. Suitable experimental procedures are described in U.S. Patent No. 3,821,383 and the references cited therein.

The following Examples illustrate the invention and the manner in which it may be performed. The preparation of starting materials is described in the designated Preparations.

In these Examples, no effort was made to optimize the yields of a given reaction. All nuclear magnetic resonance data (NMR) are in standard notation and are reported in parts per million (ppm) downfield from trimethylsilane. Deuterated dimethylsulfoxide (DMSO—$d_6$) was used as solvent in all Examples.

## Preparation A

4-Chloro-2-Hydroxymethylthiophenol

A solution of 5-chloro-2-mercaptobenzoic acid (40.0 g, 0.21 mol) in dry tetrahydrofuran (250 ml) was added dropwise to a slurry of lithium aluminum hydride (10.0 g, 0.26 mol) in dry tetrahydrofuran (50 ml) at 0°C. Following completion of addition, the reaction mixture was allowed to warm to room temperature, then stirred for 3 hours at said temperature. It was cooled to 0°C and ethyl acetate (40 ml) added to quench excess lithium aluminum hydride. The quenched reaction was stirred for 30 minutes then cautiously treated with water (10 ml), followed by 1N sodium hydroxide (40 ml). The aluminum salts which precipitated were filtered off, dissolved in 10% hydrochloric acid and the solution extracted with ethyl acetate. The extract was combined with the filtrate (from the aluminum filtration) and washed successively with 10% hydrogen chloride, water and brine. It was dried ($MgSO_4$) and evaporated under reduced pressure to give the title product as an oily solid; 35.0 g, 94% yield.

It was used as is in Preparation B.

Preparation B

5-Chloro-2-Thiomethylbenzaldehyde

Sodium methoxide (11.5 g, 0.21 mol) was added to a solution of 4-chloro-2-hydroxymethylthiophenol (35.0 g, 0.20 mol) in N,N-dimethylformamide (300 ml) at 0°C. The mixture was stirred for 30 minutes at 0°C and then iodomethane (15 ml, 0.24 mol) added. The mixture was stirred an additional 30 minutes and then poured into water (500 ml) and the product extracted therefrom with ether. The extract was washed successively with water and brine then dried ($MgSO_4$). Evaporation of the dried extract under reduced pressure gave a yellow solid.

The yellow solid was dissolved in methylene chloride (600 ml), manganese dioxide (250 g) added and the mixture stirred at room temperature for 6 hours. Filtration of the reaction mixture and evaporation of the filtrate under reduced pressure gave 33.0 g (89% yield) of the title product as an oily solid. It was used as is in Example 1.

Example 1

5-(5-Chloro-2-thiomethylphenyl)hydantoin

A mixture of 5-chloro-2-thiomethylbenzaldehyde (33.0 g, 0.18 mol), potassium cyanide (23.0 g, 0.36 mol), ammonium carbonate (68.0 g, 0.71 mol) and 20% aqueous ethanol (1200 ml) was heated at 60°C for 24 hours. It was then cooled and carefully poured into 10% hydrogen chloride (1000 ml) and the product extracted from the resulting mixture with ethyl acetate. The extract was washed with water, then with brine and dried ($MgSO_4$). Removal of the solvent under reduced pressure gave a partially solidified oil. Trituration of this residue with ether afforded 27.5 g (60% yield) of an off-white solid. M.P. = 183°—185°C.

Analysis:

| | | | |
|---|---|---|---|
| Calc'd for $C_{10}H_9N_2O_2SCl$: | 46.79% C; | 3.53% H; | 10.91% N |
| Found: | 46.91% C; | 3.65% H; | 10.58% N. |

MS: 256 ($M^+$), 209 (100%), 170.

The following compounds were prepared in like manner from the appropriate thiobenzaldehyde derivative of formula (IV) above:

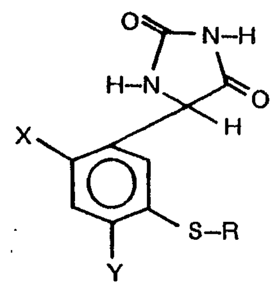

| X | Y | R | Isolation-Triturate | Calc'd / Found C | H | N | Mass Spec. (MS) | M.P. (°C) | 250 MHz NMR |
|---|---|---|---|---|---|---|---|---|---|
| H | H | $CH_3$ | 1:1ether-hexane | 54.46<br>54.04 | 4.53<br>4.56 | 12.60<br>12.53 | 222(M+, 100%), 175, 164, 136 | 137—139 | |
| H | H | $CH_2CH_3$ | 1:1ether-hexane | 55.92<br>55.86 | 5.12<br>5.06 | 11.86<br>11.75 | | 136—137 | 10.5 (bs, 1H), 8.31 (s, 1H), 7.55 (d, 2H, J=8Hz), 7.4—7.20 (M, 3H), 5.58 (s, 1H), 2.95 (q, 2H, J=8Hz), 1.21 (t, 3H, J=8Hz) |
| H | H | $(CH_2)_2CH_3$ | * | 57.58<br>57.69 | 5.64<br>5.68 | 11.19<br>11.19 | | 127—129 | 10.81 (bs, 1H), 8.30 (s, 1H), 7.53 (d, 1H, J=8Hz), 7.39—7.24 (m, 3H), 5.58 (s, 1H), 3.01—2.82 (m, 2H), 1.65—1.44 (m, 2H), 0.95 (5, 3H, J=8Hz) |
| H | H | $(CH_2)_3CH_3$ | 1:1ether-hexane | 59.07<br>59.30 | 6.10<br>5.92 | 10.59<br>10.53 | 264 (M+, 100%), 208, 207, 195, 136 | 100—103 | |
| Cl | H | $CH_2OCH_3$ | 1:1$CH_2Cl_2$-hexane | 46.08<br>46.13 | 3.87<br>3.99 | 9.77<br>9.84 | 286 (M+), 254 (100%), 211, 209, 183, 170 | 159—161 | |

| X | Y | R | Isolation-Triturate | Calc'd / Found C | H | N | Mass Spec. (MS) | M.P. (°C) | 250 MHz NMR |
|---|---|---|---|---|---|---|---|---|---|
| Cl | H | $CH_2$-4-$ClC_6H_4$ | 4:1ether-hexane | 52.32<br>52.55 | 3.29<br>3.31 | 7.62<br>7.49 | 366 (M+), 241, 170, 125 (100%) | 139—140 | |
| Cl | H | $(CH_2)_2$-4-$ClC_6H_4$ | 1:1$CH_2Cl_2$-hexane | 53.55<br>53.64 | 3.70<br>3.86 | 7.34<br>7.57 | | 184—186 | 10.92 (bs, 1H), 8.37 (s, 1H), 7.62 (d, 1H, J=8Hz), 7.45 (d, J=8Hz), 7.40—7.30 (m, 3H), 7.25 (d, 2H, J=8Hz), 5.55 (s, 1H, 3.32—3.13 (m, 2H), 2.90—2.70 (m, 2H) |
| Cl | $CF_3$ | $CH_3$ | i-propylether | 40.67 | 2.67 | 8.41 | | 244—245 | 11.05 (bs, 1H), 8.40 (s, 11H), 7.94 (s, 1H), 7.75 (s, 1H), 6.09 (s, 1H), 2.40 (s, 3H) |

* Chromatography on silica gel, elution with 60:40 hexane-ethyl acetate.

Example 2

5-(5-Chloro-2-Methylsulfinylphneyl)hydantoin

To a solution of 5-(5-chloro-2-thiomethylphenyl)-hydantoin (1.0 g, 3.9 mmol) in ethanol (50 ml) was added water (8 ml) and sodium periodate (1.7 g, 7.8 mmol) at room temperature. The mixture was stirred overnight then poured into water (50 ml) and the product extracted therefrom with ethyl acetate. The extract was dried over $MgSO_4$ and evaporated at reduced pressure to give 0.4 g (37% yield) of a white solid comprising a mixture of diastereomeric sulfoxides. M.P. 165°—170°C.

Similarly, the following compounds were prepared from the corresponding 5-(2-(RS)-phenyl)hydantoins of formula (V) above:

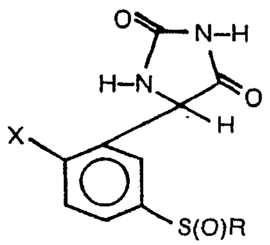

| X | R | Isolation-Trituration | Calc'd / Found % C | % H | % N | IR ($cm^{-1}$) S>O Stretch | MP (°C) |
|---|---|---|---|---|---|---|---|
| H | $CH_2CH_3$ | i-propyl ether | | | | 1012 (bs) | 119—120 |
| H | $(CH_2)_2CH_3$ | i-propyl ether | | | | 1009 (br) | 116—118 |
| H | $(CH_2)_2CH_3$ | i-propyl ether | | | | 1014 (br) | 125—130 |
| H | $CH(CH_3)_2$ | i-propyl ether | | | | 1013 (br) | 127—130 |
| Cl | $CH_2$-4-$ClC_6H_4$ | 1:1 water-acetic acid | | | | 1016 (br) | 165—170 |
| Cl | $(CH_2)_2$-4-$ClC_6H_4$ | i-propyl ether | 51.40<br>51.25 | 3.55<br>3.77 | 7.05<br>7.25 | 1015 (br) | 140—144 |

Example 3

5-(5-Chloro-2-Methylsulfonylphenyl)hydantoin

Potassium permanganate (11.0 g, 0.07 mol) and water (10 ml) were added to a slurry of 5-(5-chloro-2-thiomethylphenyl)hydantoin (9.0 g, 0.035 mol in glacial acetic acid (100 ml) at 0°C. The reaction mixture was stirred at 0°C for a half hour then poured into 500 ml of a 10% solution of sodium bisulfite in water. The product was extracted therefrom with ethyl acetate, the extract washed with water and brine, then dried ($MgSO_4$) and evaporated under reduced prssure. The white solid residue was triturated with ether, filtered and air dried. Yield = 8.5 g (84%) of title product; m.p. 238—240°C.

Analysis:

Calcd. for $C_{10}H_9N_2O_4SCl$; 41.60% C; 3.14% H; 9.70% N.

Found: 41.37% C; 3.39% H; 9.43% N.

MS: 288, 289, 209 (100%), 202.

The following compounds are made in like manner from appropriate 5-(2-(RS)-phenyl)-hydantoins of formula (V):

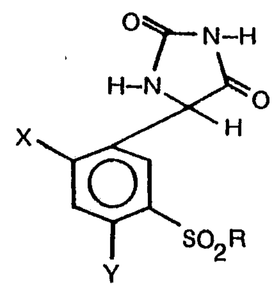

| X | Y | R | Isolation-Trituration | Calc'd / Found % C | Calc'd / Found % H | Calc'd / Found % N | MP (°C) | 250 MHz NMR |
|---|---|---|---|---|---|---|---|---|
| Cl | H | $CH_2CH_3$ | i-propyl ether | 43.64<br>43.75 | 3.66<br>3.78 | 9.25<br>8.93 | 218—219 | 11.11 (bs, 1H), 8.53 (s, 1H), 8.00 (d, 1H, J=8Hz), 7.77 (d, 1H, J=8Hz), 7.59 (s, 1H), 6.23 (s, 1H), 3.63—3.46 (m, 2H), 1.18 (t, 3H, J=8Hz) |
| Cl | H | $(CH_2)_2CH_3$ | i-propy ether | 45.50<br>45.23 | 4.14<br>4.19 | 8.84<br>8.65 | 188—189 | 11.10 (bs, 1H), 8.55 (s, 1H), 8.00 (d, 1H, J=8Hz), 7.77 (d, 1H, J=8Hz), 7.59 (s, 1H), 6.22 (s, 1H), 3.62—3.44 (m, 2H), 1.78—1.42 (m, 2H), 0.95 (t, 3H, J=8Hz) |
| Cl | H | $CH(CH_3)_2$ | i-propyl ether | 45.50<br>45.58 | 4.14<br>4.21 | 8.84<br>8.49 | 221—222 | 11.10 (bs, 1H), 8.57 (s, 1H), 7.98 (d, 1H, J=8Hz), 7.78 (d, 1H, J=8Hz), 7.60 (s, 1H), 6.19 (s, 1H) 3.83—3.67 (m, 1H), 1.32 (d, 3H, J=8Hz) |
| F | H | $CH_3$ | i-propyl ether | 44.12<br>44.12 | 3.33<br>3.49 | 10.29<br>9.89 | 222—224 | 11.10 (bs, 1H), 8.53 (s, 1H), 8.18—8.00 (m, 1H), 7.58—7.40 (m, 2H), 6.34 (s, 1H), 3.45 (s, 3H) |

| X | Y | R | Isolation-Trituration | Calc'd Found % C | % H | % N | MP (°C) | 250 MHz NMR |
|---|---|---|---|---|---|---|---|---|
| F | | $CH_2CH_3$ | ether | | | | 183—186 | 11.08 (bs, 1H), 8.55 (s, 1H), 8.10—8.04 (m, 1H), 7.60—7.38 (m, 2H), 6.25 (s, 1H), 3.60—3.48 (m, 2H), 1.16 (t, 3H, J=8Hz) |
| F | | $(CH_2)_2CH_3$ | * | 47.99<br>47.94 | 4.33<br>4.34 | 9.32<br>9.28 | 164—166 | 11.05 (bs, 1H), 8.50 (s, 1H), 8.06—8.00 (m, 1H), 7.52—7.35 (m, 2H), 6.20 (s, 1H), 3.56—3.40 (m, 2H), 1.75—1.39 (m, 2H), 0.90 (t, 3H, J=8Hz) |
| Cl | H | $CH_2$-4-$ClC_6H_4$ | 1:1 water-acetic acid | | | | 165—167 | |
| Cl | H | $(CH_2)_2$-4-$ClC_6H_4$ | water | | | | 229—231 | 11.11 (bs, 1H), 8.56 (2, 1H), 8.00 (d, 1H, J=8Hz), 7.74 (d, 1H, J=8Hz), 7.60 (s, 1H), 7.31 (s, 4H), 6.27 (s, 1H), 4.00—3.70 (m, 2H), 3.10—2.75 (m, 2H) |
| Cl | Cl | $CH_3$ | ether | | | | 225—227 | 11.05 (bs, 1H), 8.35 (s, 1H), 8.13 (s, 1H), 7.62 (s, 1H), 6.81 (s, 1H), 3.58 (s, 3H) |
| Cl | $CH_3$ | $CH_3$ | ether | 43.64<br>43.72 | 3.66<br>3.77 | 9.25<br>9.14 | 258—259 | 11.02 (bs, 1H), 8.40 (s, 1H), 7.66 (s, 1H), 7.40 (s, 1H), 6.72 (s, 1H), 3.45 (s, 3H), 2.70 (s, 3H) |
| Cl | $CF_3$ | $CH_3$ | i-propy ether | | | | 231—233 | 11.13 (bs, 1H), 8.52 (s, 1H), 8.20 (s, 1H), 7.95 (s, 1H), 6.61 (s, 1H), 3.57 (s, 3H) |

*Chromatography on silica gel, elution with 60—40 hexane-ethyl acetate.

Example 4

5-(5-Chloro-2-chlorosulfonylphenyl)hydantoin

A solution of 5-(5-chloro-2-thiomethoxymethylphenyl)hydantoin (3.5 g, 0.012 mol) in dioxane (175 ml) and water (50 ml) was cooled to 0°C. Chlorine gas was passed through the solution at 0°C until a yellow color persisted. The mixture was strred for 15 minutes then poured into 200 ml of ice cold 10% aqueous sodium bisulfite solution. The product was extracted from the resulting solution with ethyl acetate, the extract washed with water, then the brine, and dried ($MgSO_4$). Evaporation under reduced pressure gave a white solid. Trituration of the solid in ether:hexane (1:1) followed by filtration gave 2.8 g (74%) yield; M.P. 211—212°C.

250 MHz NMR: 10.92 (bs), 8.00 (bs, 1H), 7.80 (d, 1H, J=8Hz), 7.43 (d, 1H, J=8Hz), 7.12 (s, 1H), 6.20 (s, 1H).

Example 5

5-(5-Chloro-2-sulfonamidophenyl)hydantoin

Ammonia gas was passed into a slurry of 5-(5-chlorosulfonylphenyl)hydantoin (5.0 g, 0.016 mol) in methylene chloride (100 ml) at 0°C until the mixture became homogeneous and yellow in color. Stirring at 0°C was continued for one hour after which the reaction mixture was poured into 10% hydrogen chloride (200 ml) and the product extracted therefrom with ethyl acetate. The extract was washed successively with water, 10% hydrogen chloride, water and brine and then dried ($MgSO_4$). Evaporation of the dried extract under reduced pressure followed by trituration of the residue with ethyl acetate gave 2.7 g (59%) of a white solid. M.P. 239°—240°C.

Analysis:

| | | | |
|---|---|---|---|
| Calcd. for $C_9H_8N_3O_4SCl$: | 37.31% C; | 2.78% H; | 14.49% N |
| Found: | 37.62% C; | 2.99% H; | 14.11% N |

250 MHz NMR: 11.08 (bs, 1H), 8.44 (s, 1H), 7.97 (d, 1H, J=8Hz), 7.69 (d, 1H, J=8Hz), 7.63 (bs, 2H), 7.46 (s, 1H), 6.13 (s, 1H).

The following compounds are similarly prepared from the appropriate amine and 5-(2-chlorosulfonyl)hydantoin of formula (IX) above:

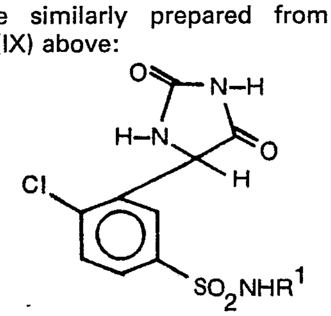

| $R^1$ | Isolation-Trituration | Calc'd / Found % C | % H | % N | MP (°C) | 250 MHz NMR |
|---|---|---|---|---|---|---|
| $CH_3$ | 1:1 ether-hexane | 39.55<br>39.61 | 3.32<br>3.38 | 13.83<br>13.61 | 205—207 | 11.09 (bs, 1H); 8.50 (s, 1H); 7.95 (d, 1H, J=8Hz), 7.69 (d, 1H, J=8Hz), 7.61 (bs, 1H), 7.50 (s, 1H), 6.08 (s, 1H), 2.49 (s, 3H) |
| $(CH_2)_2CH_3$ | ether | | | | 162—163 | 11.01 (bs, 1H), 8.42 (s, 1H), 7.90 (d, 1H, J=8Hz), 7.67—7.58 (m, 2H), 7.45 (s, 1H), 6.06 (s, 1H), 2.73 (t, 2H, J=8Hz), 1.48—1.31 (m, 2H), 0.78 (t, 3H, J=8Hz) |
| $(CH_2)_3CH_3$ | ether | 45.16<br>45.37 | 4.66<br>4.74 | 12.14<br>11.82 | 169—171 | 11.06 (bs, 1H), 8.50 (s, 1H), 7.93 (d, 1H, J=8Hz), 7.70—7.60 (m, 2H), 7.50 (s, 1H), 6.12 (s, 1H), 2.82 (q, 2H, J=8Hz), 1.48—1.20 (m, 4H), 0.84 (t, 3H, J=8Hz) |
| $(CH_2)_4CH_3$ | ether | | | | 146—148 | 11.04 (bs, 1H), 8.46 (s, 1H), 7.93 (d, 1H, J=8Hz), 7.70—7./58 (m, 2H), 7.46 (s, 1H), 6.10 (s, 1H), 2.80 (q, 2H, J=5Hz), 1.50—1.16 (m, 6H), 0.81 (t, 3H, J=5Hz) |

| $R^1$ | Isolation-Trituration | Calc'd / Found % C | % H | % N | MP (°C) | 250 MHz NMR |
|---|---|---|---|---|---|---|
| $CH_2CH_2OH$ | i-propyl ether | | | | 156—158 | 11.06 (bs, 1H), 8.45 (s, 1H), 7.95 (d, 1H, J=8Hz), 7.82 (t, 1H, J=6Hz), 7.67 (d, 1H, J=8Hz), 7.49 (s, 1H), 6.10 (s, 1H), 3.40 (t, 2H, J=7Hz), 2.88 (q, 2H, J=7Hz) |
| $(CH_2)_3OH$ | $CHCl_3$ | | | | 145 (foam) | 11.05 (s, 1H), 8.49 (s, 1H), 7.92 (d, 1H, J=8Hz), 7.71—6.63 (m, 2H), 7.49 (s, 1H), 6.09 (s, 1H), 4.46 (t, 1H, J=5Hz), 3.42—3.35 (m, 2H), 2.95—2.80 (m, 2H): 1.58 (quintet, q, 2H, J=6Hz) |
| $(CH_2)_4OH$ | i-propyl ether | 43.16<br>42.94 | 4.46<br>4.47 | 11.61<br>11.23 | | 11.05 (s, 1H), 8.48 (s, 1H), 7.93 (d, 1H, J=8Hz), 7.71—7.62 (m, 2H), 7.48 (s, 1H), 6.10 (s, 1H) 4.40 (t, 1H, J=4Hz), 3.40—3.20 (m, 2H, 2.81 (q, 2H, J=5Hz), 1.50—1.30 (m, 4H) |
| $CH_2CH_2N(CH_3)_2$ | i-propyl ether | | | | 190° (foam) | 11.00 (bs, 1H), 9.00—8.20 (b, M, 2H), 7.95 (d, 1H, J=8Hz), 7.68 (d, 1H, J=8Hz), 7.45 (s, 1H), 6.03 (s, 1H), 3.02—2.86 (m, 2H), 2.43—2.20 (m, 2H), 2.05 (s, 6H) |
| $CH_2—C_6H_5$ | * | 50.60<br>50.60 | 3.66<br>3.81 | 11.06<br>10.89 | 122—124 | 11.07 (bs, 1H), 8.48 (s, 1H), 8.25 (bs, 1H), 7.92 (d, 1H, J=8Hz), 7.63 (d, 1H, J=8Hz), 7.50 (s, 1H), 7.35—7.25 (m, 5H), 6.14 (s, 1H), 4.05 (s, 2H) |
| $(CH_2)_2—C_6H_5$ | * | | | | 177—178 | 11.05 (bs, 1H), 8.45 (s, 1H), 7.95 (d, 1H, J=8Hz), 7.75 (bs, 1H), 7.58 (d, 1H, J=8Hz), 7.48 (s, 1H), 7.30—7.13 (m, 5H), 6.17 (s, 1H), 3.15—3.00 (m, 2H), 2.76 (t, 1H, J=7Hz) |
| $(CH_2)_3—C_6H_5$ | * | | | | 145—147 | 11.06 (bs, 1H), 8.50 (s, 1H), 7.92 (d, 1H, J=8Hz), 7.78 (t, 1H, J=4Hz), 7.67 (d, 1H, J=8Hz), 7.50 (s, 1H), 7.30—7.10 (m, 5H), 6.12 (s, 1H), 2.84 (q, 2H, J=5Hz), 2.63—2.50 (m, 2H), 1.71 (quintet, 2H, J=5Hz) |
| $(CH_2)_4—C_6H_5$ | ether | 54.09<br>53.85 | 4.78<br>4.80 | 9.95<br>9.85 | 151—153 | 11.05 (bs, 1H), 8.48 (s, 1H), 7.93 (d, 1H, J=8Hz), 7.74—7.64 (m, 2H), 7.49 (s, 1H), 6.10 (s, 1H), 2.90—2.80 (m, 2H), 2.53 (t, 2H, J=5Hz), 1.62—1.36 (m, 4H) |

| $R^1$ | Isolation-Trituration | Calc'd / Found % C | % H | % N | MP (°C) | 250 MHz NMR |
|---|---|---|---|---|---|---|
| $4\text{-}FC_6H_4$ | $CHCl_3$ | 46.95<br>46.87 | 2.89<br>3.02 | 10.94<br>10.78 | 244—245 | 11.09 (bs, 1H), 10.40 (bs, 1H), 8.54 (s, 1H), 7.7 (d, 1H, J=8Hz), 7.59 (d, 1H, J=8Hz), 7.49 (s, 1H), 7.20—7.05 (m, 4H), 6.13 (s, 1H) |
| $CH_2$-2-furyl | * | | | | 169—170 | 11.02 (bs, 1H), 8.45 (s, 1H), 8.30 (bs, 1H), 7.93 (d, 1H, J=8Hz), 7.66 (d, 1H, J=8Hz), 7.58—7.54 (m, 1H), 7.48 (s, 1H), 6.39—6.35 (m, 1H), 6.25 (d, 1H, J=2Hz), 6.14 (s, 1H), 4.10 (s, 1H) |

* Chromatography on silica gel, elution with 60:40 hexane:ethyl acetate.

Example 6

5-(2-Hexylsulfonylphenyl)hydantoin

To a solution of 5-(5-chloro-2-hexylsulfinylphenyl)hydantoin (4.8 g, 0.014 mol) in glacial acetic acid (50 ml) was added potassium permanganate (2.2 g, 0.014 mol) and water (5 ml). The mixture was stirred at room temperature for 45 minutes then poured into 10% sodium bisulfite solutioin (50 ml). The resulting solution was extracted with ether, the ethereal extract then dried ($MgSO_4$) and evaporated under reduced pressure. The brown oily residue was chromatographed on silica gel and the product eluted with ethyl acetate;hexane (40:60). Evaporation of the eluate under reduced pressure afforded 1.32 g of product as a white solid (26% yield). M.P. 188—189°C.

Analysis:

Calc'd for $C_{15}H_{19}N_2O_4SCl$: 50.21% C; 5.34% H; 7.81% N

Found: 50.05% C; 5.23: H; 7.76% N

The following compounds were prepared in like manner from the corresponding 5-(2-alkylsulfinylphenyl)hydantoins:

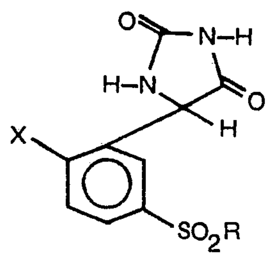

| X | R | Calc'd / Found % C | % H | % N | MP (°C) | 250 MHz NMR |
|---|---|---|---|---|---|---|
| H | $CH_2CH_3$ | | | | 185 (foam) | 11.03 (bs, 1H), 8.54 (s, 1H), 7.98 (d, 1H, J=8Hz), 7.83 (t, 1H, J=8Hz), 7.66 (t, 1H, J=8Hz), 7.55 (d, 1H, J=8Hz), 6.25 (s, 1H), 3.50 (q, 2H, J=8Hz), 1.15 (t, 3H, J=8Hz) |
| H | $(CH_2)_2CH_3$ | | | | 190 (foam) | 11.01 (bs, 1H), 8.55 (s, 1H), 7.98 (d, 1H, J=8Hz), 7.81 (t, 1H, J=8Hz), 7.65 (t, 1H, J=8Hz), 7.56 (d, 1H, J=8Hz), 6.23 (s, 1H), 3.55—3.42 (m, 2H), 1.80—1.42 (m, 2H), 0.94 (t, 3H, J=8Hz) |
| H | $CH(CH_3)_2$ | | | | 195 (foam) | 11.03 (bs, 1H), 8.55 (s, 1H), 7.98 (d, 1H, J=8Hz), 7.81 (t, 1H, J=8Hz), 7.66 (t, 1H, J=8Hz), 7.56 (d, 1H, J=8Hz), 6.18 (s, 1H), 3.71 (septet, 1H), 1.30 (d, 3H, J=8Hz), 1.09 (d, 3H, J=8Hz) |

| X | R | Calc'd / Found % C | % H | % N | MP (°C) | 250 MHz NMR |
|---|---|---|---|---|---|---|
| Cl | $(CH_2)_3CH_3$ | 47.20<br>47.10 | 4.57<br>4.61 | 8.47<br>8.42 | 191—192 | 11.09 (bs, 1H), 8.55 (s, 1H), 8.00 (d, 1H, J=8Hz), 7.77 (d, 1H, J=8Hz), 7.59 (s, 1H), 6.23 (s, 1H), 3.55 (t, 2H, J=8Hz), 1.76—1.30 (m, 4H), 0.85 (t, 3H, J=8Hz) |
| Cl | $(CH_2)_4CH_3$ | 48.77<br>48.82 | 4.97<br>4.99 | 8.12<br>8.12 | 160—161 | 11.10 (bs, 1H), 8.55 (s, 1H), 8.00 (d, 1H, J=8Hz), 7.76 (d, 1H, J=8Hz), 7.60 (s, 1H), 6.25 (s, 1H), 3.55 (t, 2H, J=9Hz), 1.78—.118 (m, 6H), 0.84 (t, 3H, J=8Hz) |

Preparation C

3-Chloro-5-Fluoro-2-Methoxybenzaldehyde

To a solution of sodium hydroxide (50 g, 1.25 mol) in water (70 ml) was added 2-chloro-4-fluorophenol (10 g, 0.068 mol) and chloroform (30 ml) and the mixture heated at reflux for two hours. An additional 30 ml of chloroform was added and refluxing continued for two more hours. This step was repeated once again after which the mixture was cooled to room temperature. The brown precipitate which formed was removed by filtration, then suspended in water and the suspension acidified with 3N HCl. The resulting tan solid, 3-chloro-5-fluoro-2-hydroxybenzaldehyde, was filtered, washed with water and dried; 4.03 g (34%). M.P. 31°—83°C.

The title compound was made by dissolving 3-chloro-5-fluoro-2-hydroxybenzaldehyde (1.25 g, 7.2 mmol) in acetone (25 ml) and adding potassium carbonate (1.27 g, 9.2 mmol) and methyl iodide (1.3 g, 9.2 mmol). The mixture was stirred overnight at room temperature then filtered. The filter cake was washed with acetone and the combined filtrate and wash concentrated under reduced pressure to a gummy solid. The solid was partitioned between methylene chloride and water. The methylene chloride phase was separated, washed successively with water, 1N NaOH and brine, then dried ($MgSO_4$) and concentration under reduced pressure. The pale yellow solid obtained (710 mg, 52% yield) melted at 59—61°C.

Preparation D

5-Chloro-2-Methoxy-3-Methylbenzaldehyde

Titanium tetrachloride (24.3 g, 0.128 mole) is added to a solution of 4-chloro-2-methylanisole (10 g, 0.064 mol) in methylene chloride (150 ml) at (8.04 g, 0.070 mol) was then added dropwise with stirring over a three minute period at 0°C. The mixture was stirred for 30 minutes at 0°C then poured into a saturated aqueous solution of sodium bicarbonate (700 ml). The organic layer was then separated and the aqueous phase extracted with methylene chloride. The combined organic extracts were washed with brine, dried ($MgSO_4$) and concentrated *in vacuo*. The residue was chromatographed on a silica gel (300 g) column using hexane-ether (6—1) as eluent. Fractions of 15 ml each were collected. Fractions 21—50 were combined and evaporated *in vacuo* to a white solid (8.9 g). This solid was rechromatographed on silica gel (300 g), eluting with hexane (15 ml fraction). Fractions 174—215 were combined and evaporated *in vacuo* to give 2.1 g of the title product as a white solid.

NMR: 2.3 (s, 3H), 3.9 (s, 3H), 7.4 (d, 1H), 7.6 (d, 1H), 10.4 (CHO).

Example 7

5-(5-Chloro-2-methoxy-3-methylphenyl)hydantoin

A mixture of 5-chloro-2-methoxy-3-methylbenzaldehyde (1.22 g, 0.0066 mol), potassium cyanide (0.86 g, 0.014 mol), ammonium carbonate (2.53 g, 0.0264 mol) and 50% aqueous ethanol (150 ml) was heated at 60°C for 5 hours. Approximately half the solvent was evaporated off under reduced pressure and the residue cooled and carefully acidified with 1*N* hydrochloric acid. The pale yellow crystals which precipitated were separated by filtration, washed with water and air dried. Yield = 1.27 g (76%). Recrystallization from ethanol-water (5:40) gave 2.03 g of white crystals; 0.086 g (51%). M.P. 182°—184°C.

Infrared (KBr, $cm^{-1}$): 3299 (m, s), 1771 (s), 1729 (s).

Examples 8—10

The following compounds were prepared according to the procedure of Example 7 from appropriate 2-methoxybenzaldehydes:

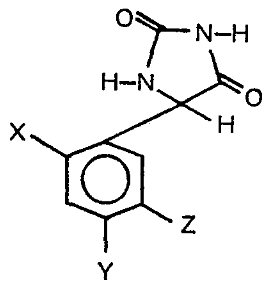

| Example | X | Y | Z | MP (°C) | IR ($cm^{-1}$) (KBr) |
|---|---|---|---|---|---|
| 8 | F | Cl | $OCH_3$ | 199—201 | 1778, 1771 |
| 9 | Cl | $CH_3$ | $OC_2H_5$ | 173—5 | 1769, 1731 |
| 10 | F | Cl | $OC_2H_5$ | 181—4 | 1771, 1720 |

**Claims for the Contracting States: BE CH DE FR GB IT LI LU NL SE**

1. A 5-(substituted phenyl)hydantoin compound having the general formula:

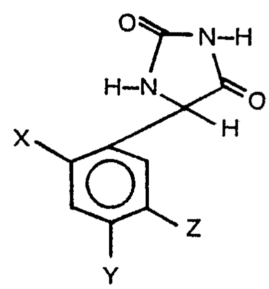

I

or a pharmaceutically acceptable salt thereof,

wherein each of X and Y is hydrogen, fluoro, chloro, bromo, ($C_1$—$C_6$)alkyl, ($C_1$—$C_6$)alkoxy, trifluoromethyl, amino or nitro;

Z is $S(O)_mR$, $SO_2NHR^1$ or ($C_1$—$C_4$)alkoxy; with the proviso that each of X and Y is fluoro, chloro, ($C_1$—$C_4$)alkyl or nitro when Z is ($C_1$—$C_4$)alkoxy;

R is ($C_1$—$C_6$)alkyl, chloro, methoxymethyl or

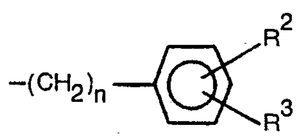 ;

m is 0, 1 or 2, with the proviso that when R is chloro, m is 2;

$R^1$ is hydrogen, furfuryl,

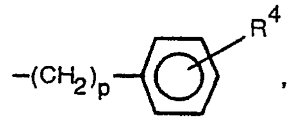 ,

($C_1$—$C_6$)alkyl, omega-substituted ($C_2$—$C_6$)alkyl wherein the substituent is hydroxy or dimethylamino;

$R^4$ is hydrogen, fluoro or chloro;

n is an integer of from 1 to 4; p is 0 or an integer of from 1 to 4;

and each of $R^2$ and $R^3$ is hydrogen, fluoro, chloro, bromo, ($C_1$—$C_6$)alkyl, ($C_1$—$C_6$)alkoxy, amino or nitro.

2. A compound according to claim 1, wherein Z is $SO_2R$, wherein R is ($C_1$—$C_6$)alkyl or

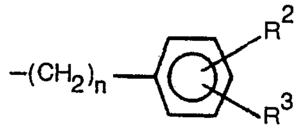

wherein n is 1 or 2 and $R^2$ and $R^3$ are as defined in claim 1.

3. A compound according to claim 2, wherein Z is $SO_2CH_3$, X is fluoro or chloro and Y is fluoro, chloro, methyl or hydrogen.

4. A compound according to claim 2, wherein Z is

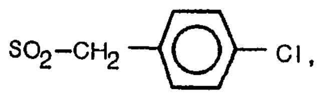

X is fluoro or chloro and Y is hydrogen.

5. A compound according to claim 1, wherein Z is $SO_2NHR^1$, wherein $R^1$ is furfuryl or

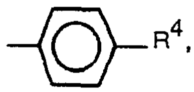

wherein $R^4$ is fluoro or chloro, X is fluoro or chloro and Y is hydrogen, fluoro, chloro or methyl.

6. A compound according to claim 1, wherein Z is $SCH_3$ or $SCH_2OCH_3$, X is chloro or fluoro; and Y is hydrogen, fluoro, chloro, methyl or trifluoromethyl.

7. A compound according to claim 1, wherein Z is $SO_2Cl$, X is chloro or fluoro and Y is hydrogen, chloro, fluoro or methyl.

8. A compound according to claim 1, wherein Z is methoxy or ethoxy, X is fluoro or chloro and Y is fluoro, chloro or methyl.

9. A pharmaceutical composition for the treatment of diabetes associated complications, which comprises an effective amount of a compound according to claim 1 in admixture with a pharmaceutically-acceptable carrier or diluent.

10. A compound according to claim 1, for use in the treatment of diabetes associated complications.

**Claims for the Contracting State: AT**

1. A process for the preparation of a 5-(substituted phenyl)hydantoin compound having the general formula:

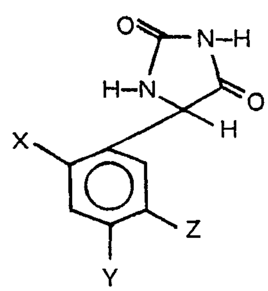

I

or a pharmaceutically acceptable salt thereof,

wherein each of X and Y is hydrogen, fluoro, chloro, bromo, $(C_1—C_6)$alkyl, $(C_1—C_6)$alkoxy, trifluoromethyl, amino or nitro;

Z is $S(O)_mR$, $SO_2NHR^1$ or $(C_1—C_4)$alkoxy, with the proviso that each of X and Y is fluoro, chloro, $(C_1—C_4)$alkyl or nitro when Z is $(C_1—C_4)$alkoxy;

R is $(C_1—C_6)$alkyl, chloro, methoxymethyl or

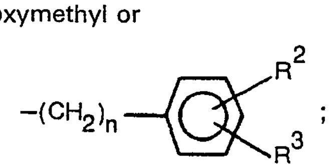

m is 0, 1 or 2, with the proviso that when R is chloro, m is 2;

$R^1$ is hydrogen, furfuryl,

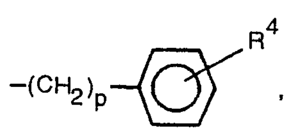

$(C_1—C_6)$alkyl, omega-substituted $(C_2—C_6)$alkyl wherein the substituent is hydroxy or dimethylamino;

$R^4$ is hydrogen, fluoro, or chloro;

n is an integer of from 1 to 4; p is 0 or an integer of from 1 to 4;

and each of $R^2$ and $R^3$ is hydrogen, fluoro, chloro, bromo, ($C_1$—$C_6$)alkyl, ($C_1$—$C_6$)alkoxy, amino or nitro, characterized in that:—

(1) when Z is —SR or ($C_1$—$C_4$)alkoxy a 2-mercaptobenzaldehyde of the formula:—

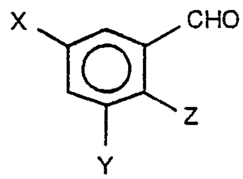

wherein Z is SR or ($C_1$—$C_4$)alkoxy and X, Y and R are as defined above, is reacted with ammonium carbonate and potassium or sodium cyanide in aqueous alcohol solution at a temperature of from 50°C. to 60°C.;

(2) when Z is —S(O)R, a hydantoin thioether of the formula:—

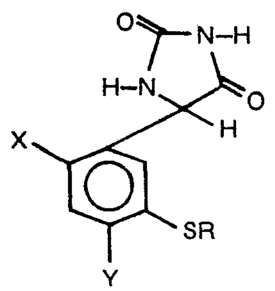

(V)

as formed in step (1), is reacted with sodium periodate in aqueous alcoholic solution at ambient temperature using a 1:2 molar ratio of thioether to sodium periodate;

(3) when Z is —$SO_2R$, a hydantoin thioether of formula (V) is reacted with potassium permanganate in acetic acid at a temperature of from −10°C. to room temperature;

(4) when Z is $SO_2Cl$, an hydantoin thioether of formula (V) wherein R is methoxymethyl, is reacted with chlorine in a reaction-inert solvent;

(5) when Z is $SO_2NHR^1$, a sulfonyl chloride of the formula:

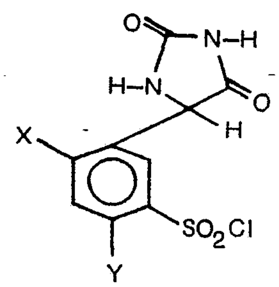

(VIII)

as formed in step (4), is reacted with an amine of formula $H_2NR^1$ in a reaction-inert solvent at a temperature of from 0°C. to 50°C.; and

(6) if desired, the compound of formula (I) is converted into a pharmaceutically-acceptable salt thereof.

2. A process according to claim 1, wherein Z is $SO_2R$, wherein R is ($C_1$—$C_6$)alkyl or

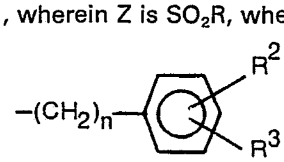

wherein n is 1 or 2 and $R^2$ and $R^3$ are as defined in claim 1.

3. A process according to claim 2, wherein Z is $SO_2CH_3$, X is fluoro or chloro and Y is fluoro, chloro, methyl or hydrogen.

4. A process according to claim 2, wherein Z is

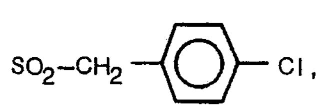

X is fluoro or chloro and Y is hydrogen.

5. A process according to claim 1, wherein Z is $SO_2NHR^1$, wherein $R^1$ is furfuryl or

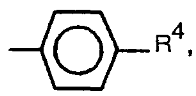

wherein $R^4$ is fluoro or chloro, X is fluoro or chloro and Y is hydrogen, fluoro, chloro or methyl.

6. A process according to claim 1, wherein Z is $SCH_3$ or $SCH_2OCH_3$, X is chloro or fluoro; and Y is hydrogen, fluoro, chloro, methyl or trifluoromethyl.

7. A process according to claim 1, wherein Z is $SO_2Cl$, X is chloro or fluoro and Y is hydrogen, chloro, fluoro or methyl.

8. A process according to claim 1, wherein Z is methoxy or ethoxy, X is fluoro or chloro and Y is fluoro, chloro or methyl.

**Patentansprüche für die Vertragsstaaten: BE CH DE FR GB IT LI LU NL SE**

1. 5-(Subst. Phenyl)-hydantoinverbindung der allgemeinen Formel

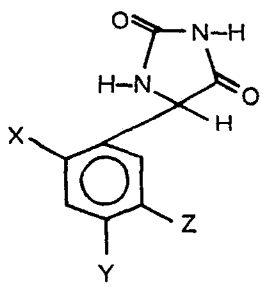

I

oder ein pharmazeutisch annehmbares Salzes hievon, worin jedes von X und Y Wasserstoff, Fluor, Chlor, Brom, ($C_1$—$C_6$)-Alkyl, ($C_1$—$C_6$)-Alkoxy, Trifluormethyl, Amino oder Nitro bedeutet; Z $S(O)_mR$, $SO_2NHR^1$ oder ($C_1$—$C_4$)-Alkoxy darstellt; mit der Maßgabe, daß jedes von X und Y Fluor, Chlor, ($C_1$—$C_4$)-Alkyl oder Nitro ist, wenn Z ($C_1$—$C_4$)-Alkoxy bedeutet; R ($C_1$—$C_6$)-Alkyl, Chlor, Methoxymethyl oder

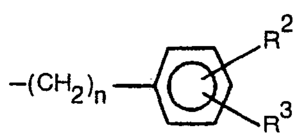

darstellt; m Null, 1 oder 2 ist, mit der Maßgabe, daß, wenn R Chlor bedeutet, m 2 ist; $R^1$ Wasserstoff, Furfuryl,

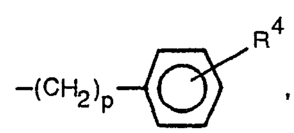

,

($C_1$—$C_6$)-Alkyl oder ω-substituiertes ($C_2$—$C_6$)-Alkyl darstellt, wobei der Substituent Hydroxy oder Dimethylamino ist; $R^4$ Wasserstoff, Fluor oder Chlor bedeutet; n eine ganze Zahl von 1 bis 4 ist; p Null oder eine ganze Zahl von 1 bis 4 ist; und jedes von $R^2$ und $R^3$ Wasserstoff, Fluor, Chlor, Brom, ($C_1$—$C_6$)-Alkyl, ($C_1$—$C_6$)-Alkoxy, Amino oder Nitro bedeutet.

2. Verbindung nach Anspruch 1, worin Z die Bedeutung $SO_2R$ hat, wobei R ($C_1$—$C_6$)-Alkyl oder

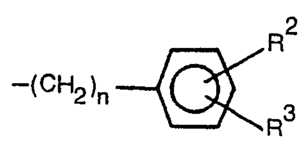

ist, worin n 1 oder 2 ist und $R^2$ und $R^3$ wie in Anspruch 1 definiert sind.

3. Verbindung nach Anspruch 2, worin Z $SO_2CH_3$, X Fluor oder Chlor und Y Fluor, Chlor, Methyl oder Wasserstoff bedeuten.

4. Verbindung nach Anspruch 2, worin Z die Bedeutung

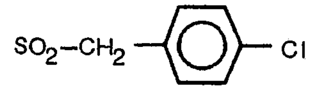

hat, X Fluor oder Chlor bedeutet und Y Wasserstoff ist.

5. Verbindung nach Anspruch 1, worin Z $SO_2NHR^1$ ist, wobei $R^1$ Furfuryl oder

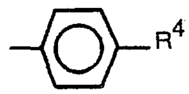

ist, wobei $R^4$ Fluor oder Chlor bedeutet, X Fluor oder Chlor darstellt und Y Wasserstoff, Fluor, Chlor oder Methyl ist.

6. Verbindung nach Anspruch 1, worin Z $SCH_3$ oder $SCH_2OCH_3$, X Chlor oder Fluor und Y Wasserstoff, Fluor, Chlor, Methyl oder Trifluormethyl bedeuten.

7. Verbindung nach Anspruch 1, worin Z $SO_2Cl$, X Chlor oder Fluor und Y Wasserstoff, Chlor, Fluor oder Methyl bedeuten.

8. Verbindung nach Anspruch 1, worin Z Methoxy oder Äthoxy, X Fluor oder Chlor und Y Fluor, Chlor oder Methyl bedeuten.

9. Pharmazeutische Zusammensetzung zur Behandlung von mit Diabetes verbundenen Komplikationen, welche einen wirksamen Anteil einer Verbindung nach Anspruch 1 in Mischung mit einem pharmazeutisch annehmbaren Träger oder Verdünnungsmittel umfaßt.

10. Verbindung nach Anspruch 1 zur Verwendung bei der Behandlung von mit Diabetes verbundenen Komplikationen.

**Patentansprüche für den Vertragsstaat: AT**

1. Verfahren zur Herstellung einer 5-(subst.Phenyl)-hydantoinverbindung der allgemeinen Formel

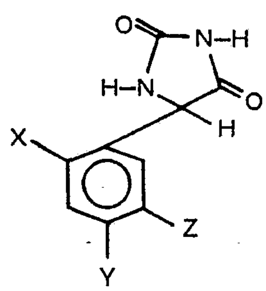

I

oder eines pharmazeutisch annehmbaren Salzes hievon, worin jedes von X und Y Wasserstoff, Fluor, Chlor, Brom, $(C_1-C_6)$-Alkyl, $(C_1-C_6)$-Alkoxy, Trifluormethyl, Amino oder Nitro bedeutet; Z $S(O)_mR$, $SO_2NHR^1$ oder $(C_1-C_4)$-Alkoxy darstellt; mit der Maßgabe, daß jedes von X und Y Fluor, Chlor, $(C_1-C_4)$-Alkyl oder Nitro ist, wenn Z $(C_1-C_4)$-Alkoxy bedeutet; R $(C_1-C_6)$-Alkyl, Chlor, Methoxymethyl oder

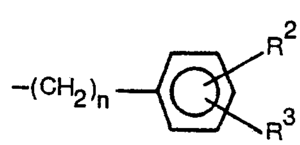

darstellt; m Null, 1 oder 2 ist, mit der Maßgabe, daß, wenn R Chlor bedeutet, m 2 ist; $R^1$ Wasserstoff, Furfuryl,

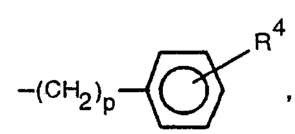

,

$(C_1-C_6)$-Alkyl oder ω-substituiertes $(C_2-C_6)$-Alkyl darstellt, wobei der Substituent Hydroxy oder Dimethylamino ist; $R^4$ Wasserstoff, Fluor oder Chlor bedeutet; n eine ganze Zahl von 1 bis 4 ist; p Null oder eine ganze Zahl von 1 bis 4 ist; und jedes von $R^2$ und $R^3$ Wasserstoff, Fluor, Chlor, Brom, $(C_1-C_6)$-Alkyl, $(C_1-C_6)$-Alkoxy, Amino oder Nitro bedeutet, dadurch gekennzeichnet, daß

(1) wenn Z —SR oder $(C_1-C_4)$-Alkoxy bedeutet, ein 2-Mercaptobenzaldehyd der Formel

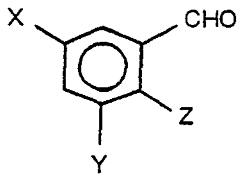

worin Z SR oder ($C_1$—$C_4$)-Alkoxy ist und X, Y und R wie oben definiert sind, mit Ammoniumcarbonat und Kalium- oder Natriumcyanid in wässeriger Alkohollösung bei einer Temperatur von 50 bis 60°C umgesetzt wird;

(2) wenn Z —S(O)R ist, ein Hydantointhioäther der Formel

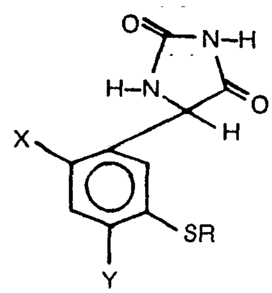

(V)

wie in Schritt (1) gebildet, mit Natriumperjodat in wässeriger alkoholischer Lösung bei Umgebungstemperatur unter Anwendung eines Molverhältnisses von Thioäther zu Natriumperjodat von 1:2 umgesetzt wird;

(3) wenn Z —$SO_2R$ ist, ein Hydantointhioäther der Formel (V) mit Kaliumpermanganat in Essigsäure bei einer Temperatur von −10°C bis Raumtemperatur umgesetzt wird;

(4) wenn Z $SO_2Cl$ bedeutet, ein Hydantointhioäther der Formel (V), worin R Methoxymethyl ist, mit Chlor in einem reaktionsinerten Lösungsmittel umgesetzt wird;

(5) wenn Z $SO_2NHR^1$ bedeutet, ein Sulfonylchlorid der Formel

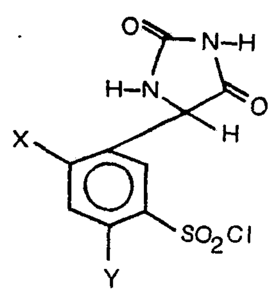

(VIII)

wie in Schritt (4) gebildet, mit einem Amin der Formel $H_2NR^1$ in einem reaktionsinerten Lösungsmittel bei einer Temperatur von 0 bis 50°C umgesetzt wird; und

(6) wenn gewünscht, die Verbindung der Formel (I) in ein pharmazeutisch annehmbares Salz überführt wird.

2. Verfahren nach Anspruch 1, worin Z die Bedeutung $SO_2R$ hat, wobei R ($C_1$—$C_6$)-Alkyl oder

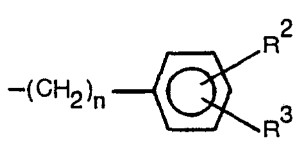

ist, worin n 1 oder 2 ist und $R^2$ und $R^3$ wie in Anspruch 1 definiert sind.

3. Verfahren nach Anspruch 2, worin Z $SO_2CH_3$, X Fluor oder Chlor und Y Fluor, Chlor, Methyl oder Wasserstoff bedeuten.

4. Verfahren nach Anspruch 2, worin Z die Bedeutung

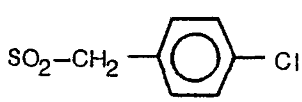

hat, X Fluor oder Chlor bedeutet und Y Wasserstoff ist.

5. Verfahren nach Anspruch 1, worin Z $SO_2NHR^1$ ist, wobei $R^1$ Furfuryl oder

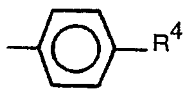

ist, wobei $R^4$ Fluor oder Chlor bedeutet, X Fluor oder Chlor darstellt und Y Wasserstoff, Fluor, Chlor oder Methyl ist.

6. Verfahren nach Anspruch 1, worin Z $SCH_3$ oder $SCH_2OCH_3$, X Chlor oder Fluor und Y Wasserstoff, Fluor, Chlor, Methyl oder Trifluormethyl bedeuten.

7. Verfahren nach Anspruch 1, worin Z $SO_2Cl$, X Chlor oder Fluor und Y Wasserstoff, Chlor, Fluor oder Methyl bedeuten.

8. Verfahren nach Anspruch 1, worin Z Methoxy oder Äthoxy, X Fluor oder Chlor und Y Fluor, Chlor oder Methyl bedeuten.

**Revendications pour les Etats contractants: BE CH DE FR GB IT LI LU NL SE**

1. 5-(phényle substitué)hydantoïne de formule générale:

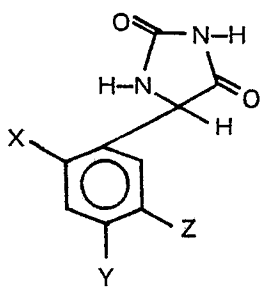

I

ou un sel pharmaceutiquement acceptible de ce composé,

formule dans laquelle chacun des groupes X et Y représente l'hydrogène, un groupe fluoro, chloro, bromo, alkyle en $C_1$ à $C_6$, alkoxy en $C_1$ à $C_6$, trifluorométhyle, amino ou nitro:

Z est un groupe $S(O)_mR$, $SO_2NHR^1$ ou alkoxy en $C_1$ à $C_4$; sous réserve que chacun de X et Y soit un groupe fluoro, chloro, alkyle en $C_1$ à $C_4$ ou nitro lorsque Z est un groupe alkoxy en $C_1$ à $C_4$;

R est un groupe alkyle en $C_1$ à $C_6$, chloro, méthoxyméthyle ou

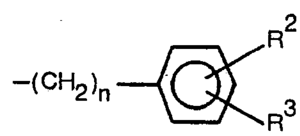

;

m a la valeur 0, 1 ou 2, sous réserve que lorsque R est un groupe chloro, m soit égal à 2;

$R^1$ est l'hydrogène, un groupe furfuryle,

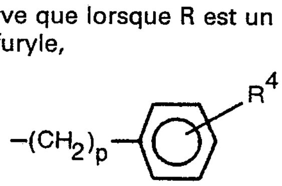

alkyle en $C_1$ à $C_6$, alkyle en $C_2$ à $C_6$ oméga-substitué dont le substituant est un radical hydroxy ou diméthylamino;

$R^4$ est l'hydrogène, un groupe fluoro ou chloro;

n est un nombre entier de 1 à 4; p est égal à O ou à un nombre entier de 1 à 4;

et chacun de $R^2$ et $R^3$ représente l'hydrogène ou un groupe fluoro, bromo, alkyle en $C_1$ à $C_6$, alkoxy en $C_1$ à $C_6$, amino ou nitro.

2. Composé suivant la revendication 1, dans lequel Z représente $SO_2R$ où R est un groupe alkyle en $C_1$ à $C_6$ ou

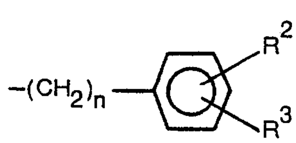

n est égal à 1 ou 2 et $R^2$ et $R^3$ sont tels que définis dans la revendication 1.

3. Composé suivant la revendication 2, dans lequel Z est un groupe $SO_2CH_3$, X est un groupe fluoro ou chloro et Y est un groupe fluoro, chloro, méthyle ou l'hydrogène.

4. Composé selon la revendication 2, dans lequel Z est le groupe

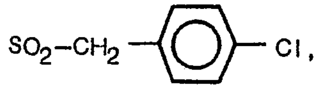

X est un groupe fluoro ou chloro et Y est l'hydrogène.

5. Composé suivant la revendication 1, dans lequel Z est un groupe $SO_2NHR^1$, dans lequel $R^1$ est un groupe furfuryle ou

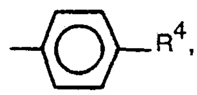

où $R^4$ est un groupe fluoro ou chloro, X est un groupe fluoro ou chloro et Y est l'hydrogène ou un groupe fluoro, chloro ou méthyle.

6. Composé suivant la revendication 1, dans lequel Z est un groupe $SCH_3$ ou $SCH_2OCH_3$, X est un groupe chloro ou fluoro; et Y est l'hydrogène ou un groupe fluoro, chloro, méthyle ou trifluorométhyle.

7. Composé suivant la revendication 1, dans lequel Z est le groupe $SO_2Cl$, X est un groupe chloro ou fluoro et Y est l'hydrogène ou un groupe chloro, fluoro ou méthyle.

8. Composé suivant la revendication 1, dans lequel Z est un groupe méthoxy ou éthoxy, X est un groupe fluoro ou chloro et Y est un groupe fluoro, chloro ou méthyle.

9. Composition pharmaceutique destinée au traitement des complications liées au diabète, qui comprend une quantité efficace d'un composé suivant la revendication 1 en mélange avec un support ou diluant pharmaceutiquement acceptable.

10. Composé suivant la revendication 1, destiné à être utilisé dans le traitement de complications liées au diabète.

**Revendications pour l'Etat contractant: AT**

1. Procédé de préparation d'une 5-(phényle substitué)hydantoïne de formule générale:

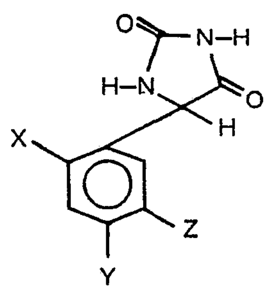

I

ou d'un sel pharmaceutiquement acceptable de ce composé,

formule dans laquelle chacun des groupes X et Y représente l'hydrogène, un groupe fluoro, chloro, bromo, alkyle en $C_1$ à $C_6$, alkoxy en $C_1$ à $C_6$, trifluorométhyle, amino ou nitro:

Z est un groupe $S(O)_mR$, $SO_2NHR^1$ ou alkoxy en $C_1$ à $C_4$; sous réserve que chacun de X et Y soit un groupe fluoro, chloro, alkyle en $C_1$ à $C_4$ ou nitro lorsque Z est un groupe alkoxy en $C_1$ à $C_4$;

R est un groupe alkyle en $C_1$ à $C_6$, chloro, méthoxyméthyle ou

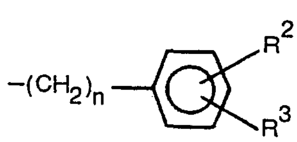

;

m a la valeur 0, 1 ou 2, sous réserve que lorsque R est un groupe chloro, m soit égal à 2;

$R^1$ est l'hydrogène, un groupe furfuryle,

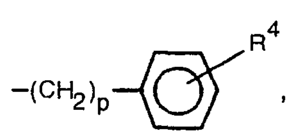

,

alkyle en $C_1$ à $C_6$ alkyle en $C_2$ à $C_6$ oméga-substitué dont le substituant est un radical hydroxy ou diméthylamino;

$R^4$ est l'hydrogene, un groupe fluoro ou chloro;

n est un nombre entier de 1 à 4; p est égal à 0 ou à un nombre entier de 1 à 4;

et chacun de $R^2$ et $R^3$ représente l'hydrogène ou un groupe fluoro, chloro, bromo, alkyle en $C_1$ à $C_6$, alkoxy en $C_1$ à $C_6$, amino ou nitro,

caractérisé en ce que:

(1) lorsque Z représente —SR ou un groupe alkoxy en $C_1$ à $C_4$, on fait réagir un 2-mercaptobenzaldéhyde de formule:

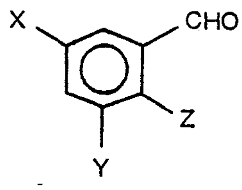

dans laquelle Z est un groupe SR ou alkoxy en $C_1$ à $C_4$ et X, Y et R sont tels que définis ci-dessus, avec le carbonate d'ammonium et le cyanure de potassium ou de sodium en solution alcoolique aqueuse à une température de 50 à 60°C;

(2) lorsque Z est un groupe —S(O)R, on fait réagir un thioéther d'hydantoïne de formule:

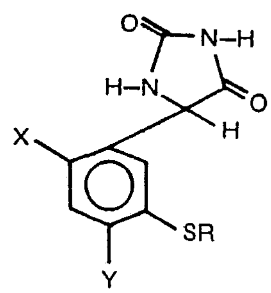

(V)

tel qu'obtenu dans l'étape (1), avec le periodate de sodium en solution alcoolique aqueuse à la température ambiante dans un rapport molaire du thioéther au periodate de sodium de 1:2;

(3) lorsque Z est un groupe $—SO_2R$, on fait réagir un thioéther d'hydantoïne de formule (V) avec le permanganate de potassium dans l'acide acétique à une température allant de −10°C à la température ambiante;

(4) lorsque Z est le groupe $SO_2Cl$, on fait réagir un thioéther d'hydantoïne de formule (V) dans laquelle R est le groupe méthoxyméthyle, avec du chlore dans un solvant inerte vis-à-vis de la réaction;

(5) lorsque Z est un groupe $SO_2NHR^1$, on fait réagir un chlorure de sulfonyle de formule:

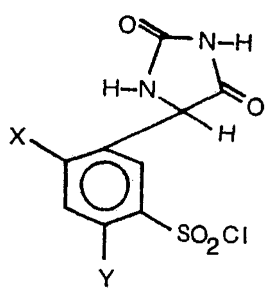

(VIII)

tel qu'obtenu dans l'étape (4), avec une amine de formule $H_2NR^1$ dans un solvant inerte vis-à-vis de la réaction à une température de 0 à 50°C; et

(6) le cas échéant, on convertit le composé de formule (I) en un sel pharmaceutiquement acceptable de ce composé.

2. Procédé suivant la revendication 1, dans lequel Z est un groupe $SO_2R$ dans lequel R est un groupe alkyle en $C_1$ à $C_6$ ou

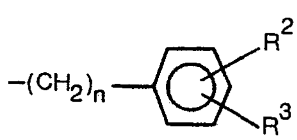

n a la valeur 1 ou 2 et $R^2$ et $R^3$ sont tels que définis dans la revendication 1.

3. Procédé suivant la revendication 2, dans lequel Z est le groupe $SO_2CH_3$, X est un groupe fluoro ou chloro et Y est un groupe fluoro, chloro, méthyle ou l'hydrogène.

4. Procédé suivant la revendication 2, dans lequel Z est le groupe

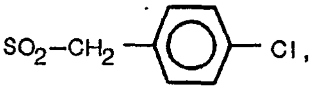

X est un groupe fluoro ou chloro et Y est l'hydrogène.

5. Procédé suivant la revendication 1, dans lequel Z est un groupe $SO_2NHR^1$ où $R^1$ est un groupe furfuryle ou

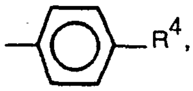

dans lequel $R^4$ est un groupe fluoro ou chloro, X est un groupe fluoro ou chloro et Y est l'hydrogène ou un groupe fluoro, chloro ou méthyle.

6. Procédé suivant la revendication 1, dans lequel Z est le groupe $SCH_3$ ou $SCH_2OCH_3$, X est un groupe chloro ou fluoro et Y est l'hydrogène ou un groupe fluoro, chloro, méthyle ou trifluorométhyle.

7. Procédé suivant la revendication 1, dans lequel Z est le groupe $SO_2Cl$, X est un groupe chloro ou fluoro et Y est l'hydrogène ou un groupe chloro, fluoro ou méthyle.

8. Procédé suivant la revendication 1, dans lequel Z est le groupe méthoxy ou éthoxy, X est un groupe fluoro ou chloro et Y est un groupe fluoro, chloro ou méthyle.